# EUROPÄISCHE PATENTSCHRIFT

(11) **EP 1 253 412 B1**
(45) Veröffentlichungstag und Bekanntmachung des Hinweises auf die Patenterteilung: **17.11.2004**
(21) Anmeldenummer: 02405304.3
(22) Anmeldetag: 15.04.2002
(51) Int. Cl.: G01L 9/00

(54) **Druckmesssystem**
System for measuring pressure
Système de mesure de pression

(30) Priorität: 25.04.2001 CH 7562001
(43) Veröffentlichungstag der Anmeldung: 30.10.2002
(73) Patentinhaber: Oertli-Instrumente AG, 9442 Berneck (CH)
(72) Erfinder: Dietrich, Andreas, 9445 Rebestein (CH)
(74) Vertreter: Liebetanz, Michael, Dipl.-Phys.

(56) Entgegenhaltungen:
- FR-A- 2 163 936
- US-A- 3 841 157
- US-A- 5 505 092
- US-A- 5 756 900

## Beschreibung

Die Erfindung betrifft ein Druckmesssystem mit einer von einem Fluid durchflossenen Leitung, wobei die Leitung einen Leitungsabschnitt aufweist, in dem auf einer abgeflachten Aussenseite der Leitung eine Membran vorgesehen ist, die unlösbar mit einer von der Leitung wegweisenden Nase verbunden ist, die über einen im wesentlichen quer zur longitudinalen Ausrichtung des Leitungsabschnittes ausgerichteten Fortsatz verfügt, dessen für Druckänderungen charakteristische Bewegung an einen Drucksensor übertragbar ist.

Eine solches Druckmesssystem ist aus der US 3,841,157 bekannt. Dabei werden zwei gegenüberliegende Fortsätze zum Einhängen der Leitung genutzt, die über Federstähle fest in dem Druckmesssystem eingespannt sind.

Ein weiteres ähnliches Druckmesssystem ist aus der FR 2,163,936 bekannt, bei dem eine Leitung zwischen den freien Enden von zwei Balken geklemmt wird, die auf ihrer jeweils anderen Seite fest zusammengeschraubt sind. In einem Balken ist ein Dehnungssensor über einen dünneren Abschnitt vorgesehen, um den sich aus der Deformation der kreisrunden Leitung ergebenden Druck durch eine Dehnungsmessung zu erfassen.

Solche Messsysteme, wie aus der US 5,392,653 bekannt, werden in Kassetten eines Aspirationssystems eingesetzt. Bei einem Aspirationssystem wird mittels einer Pumpe Flüssigkeit abgesaugt. Bei der Pumpe kann es sich beispielsweise um eine Rollenpumpe oder eine Venturi-Pumpe handeln. Unabhängig von der eingesetzten Pumpe wird aufgrund des dem Fluss entgegengebrachten Widerstandes in den Schläuchen oder Leitungen bei einer Okklusion am Schlauchende in den Schläuchen oder Leitungen ein Unterdruck entstehen. Bei üblichen Systemen verbindet man über eine T-Verzweigung das Schlauchsystem direkt mit einem Drucksensor.

Nun werden solche Aspirationssysteme beispielsweise in der Kataraktoperation (grauer Star) eingesetzt und dienen dazu, die während der Operation entstehenden Linsenfragmente zusammen mit einer Infusionsflüssigkeit abzusaugen. Die heutige Operationstechnik besteht darin, solche Linsenteile mit dem an das Aspirationssystem angeschlossene Instrument anzusaugen, festzuhalten, in eine geeignete Position zu bringen und mittels Ultraschall zu zerkleinern und anschliessend abzusaugen. Dabei entsteht notwendigerweise während einer gewissen Zeit eine Okklusion, da die Absaugöffnung durch Linsenteile verstopft wird. Es baut sich zeitlich nachfolgend im Innern des Aspirationssystems ein grösserer Unterdruck auf. Beim Lösen der Okklusion, was zumeist sehr rasch geschieht, baut sich der Unterdruck ab und zieht dabei Infusionsflüssigkeit nach, was bei der oben genannten Operation zu einem Kollabieren der Vorderkammer des zu operierenden Auges führen kann.

Dieser Nachsaug- und Kollapseffekt ist vor allem dann gross, wenn sich in der Aspirationsleitung Luft befindet. Das von der Luft unter Unterdruck eingenommene Volumen reduziert sich bei einem Brechen der Okklusion schlagartig und saugt besonders viel Flüssigkeit nach. Bei der bekannten Methode mit Einsatz einer T-Verzweigung ist ein Einschluss von Luft unumgänglich. Zudem kann bei diesem Arbeitsprinzip die Aspirationsflüssigkeit mit dem Drucksensor in Berührung kommen. Da sich dieser Drucksensor nicht sterilisieren lässt, besteht die Möglichkeit einer Kontamination des Patienten mit vom Drucksensor herrührenden Bakterien, wenn der Arzt bei der Operation rückspült, was bei gewissen Operationsschritten notwendig ist.

Mit der Vorrichtung nach der US 5,392,653 kann der Einschluss von Luft verhindert werden und ein Kontakt der im Schlauchsystem befindlichen Flüssigkeit mit einem nicht sterilisierbaren Sensorelement wird vermieden. Dafür wird auf die Druckmesskammer fest eine metallische Scheibe aufgebracht. Diese Scheibe wird mittels Magnetkraft mit einem Kraft- oder Wegsensor gekoppelt.

Dieses System weist den Nachteil auf, dass in der Druckmesskammer sehr stark vom kreisrunden Querschnitt des Leitungssystems, d.h. des Aspirationsschlauches, abweichende geometrische Verhältnisse herrschen. Es ist darum schwierig, die Kammer vollständig mit Fluid zu füllen und die abgesaugten Linsenfragmente bleiben oft in der Kammer haften. Es ist nicht möglich, dieses Schlauchsystem für die Wiederaufbereitung im Autoklav-Sterilisator einzusetzen, da nicht die Möglichkeit besteht, es vollständig zu entleeren und Linsenfragmente sicher auszuspülen.

Die US 3,841,157 weist zwar eine abgeflachte Membran auf und kann mit den beiden Einsätzen in die Vorrichtung eingehängt werden. Auch hier ist es schwierig, die Kammer vollständig mit einer Flüssigkeit zu füllen, wenn es darum geht, abgesaugte Linsenfragmente vollständig abzusaugen. Die Anordnung des Messsensors über die zwei Federstähle bei der US 3,841,157 ist der Messgenauigkeit abträglich.

Ausgehend von diesem Stand der Technik liegt der Erfindung die Aufgabe zugrunde, ein Druckmesssystem der eingangs genannten Art anzugeben, welches für die Wiederaufbereitung im Autoklav-Sterilisator einsetzbar ist und eine bessere Messgenauigkeit gestattet. Diese Aufgabe wird erfindungsgemäss für ein Druckmesssystem der eingangs genannten Art durch die kennzeichnenden Merkmale des Anspruchs 1 gelöst.

Dadurch, dass die Membran unlösbar mit einer von der Leitung wegweisenden Nase verbunden ist, die über einen im wesentlichen quer zur longitudinalen Ausrichtung des Leitungsabschnittes ausgerichteten Fortsatz verfügt, kann der Querschnitt des Leitungssystems im wesentlichen kreisrund mit den entsprechenden Spülungsvorteilen gehalten werden.

Dadurch, dass diese Nase eine Bohrung aufweist, durch die der Fortsatz in Gestalt eines Stiftes ragt, kann in einfacher Weise mechanisch und zugleich lösbar ein Kraftsensor mit den Ausschlägen der Membran beaufschlagt werden.

Weitere vorteilhafte Ausführungsformen sind in den Unteransprüchen gekennzeichnet.

Nachstehend wird ein Ausführungsbeispiel der Erfindung beispielhaft unter Bezugnahme auf die beigefügten Zeichnungen beschrieben. Es zeigen:
- Fig. 1: eine perspektivische Darstellung einer erfindungsgemässsen Anordnung.
- Fig. 2: eine Schnittzeichnung durch die Anordnung nach Fig. 1 bei normalen Druckverhältnissen,
- Fig. 3: eine Schnittzeichnung durch die Anordnung nach Fig. 1 bei Unterdruck,
- Fig. 4: eine Querschnittsansicht durch den Schlauch im Bereich des Stabes,
- Fig. 5: ein Ausführungsbeispiel einer Umsetzung der Membranbewegung nach Fig. 4, und
- Fig. 6: eine Schnittansicht des Ausführungsbeispiel nach Fig. 5.

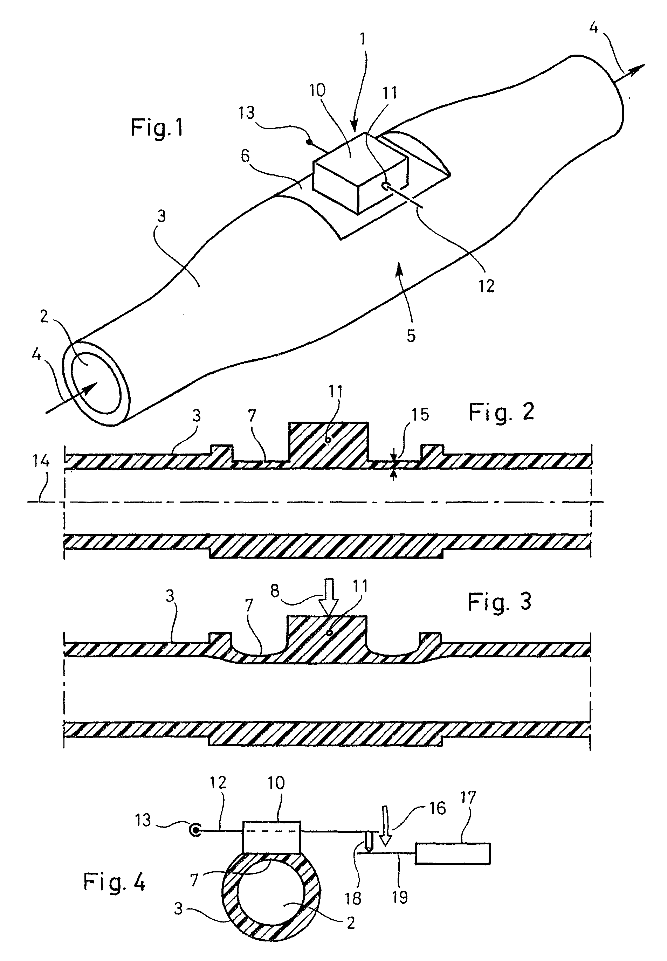

Beim Ausführungsbeispiel nach der Erfindung ist es ein Ziel, die Druckmessung mit einem Druckmesselement 1 vorzunehmen, das den kreisrunden Querschnitt 2 des Aspirationsschlauches 3 möglichst ohne Veränderung weiterführt und damit einen Einschluss von Luft eliminiert, Linsenfragmente nicht gefangen hält und insgesamt einwandfrei gespült werden kann. Die Fig. 1 zeigt eine perspektivische Darstellung einer solchen erfindungsgemässen Anordnung. Der Aspirationsschlauch 3 wird von einem durch die Pfeile 4 angedeuteten Fluid durchflossen. In der Mitte der Fig. 1 ist ein Leitungsabschnitt 5 dargestellt, in dem auf einer abgeflachten Aussenseite 6 der Leitung 3 eine in der Fig. 2 zu erkennende Membran 7 vorgesehen ist. Die Membran 7 ist unlösbar mit einer von der Leitung 3 wegweisenden Nase 10 verbunden, die bei dem in der Fig. 1 dargestellten Ausführungsbeispiel über eine Bohrung 11 verfügt, in der ein Stift 12 einsetzbar ist. Der Stift 12 ist an einem festen Punkt 13 angelenkt, so dass sich Bewegungen der Membran 7 durch Bewegungen der Nase 10 in verstärkte Verschwenkbewegungen des Stiftes 12 übersetzen.

Die Fig. 2 zeigt nun eine Schnittzeichnung durch die Anordnung nach Fig. 1 bei normalen Druckverhältnissen. Ein Schlauchstück, der Schlauchabschnitt 5 wird mit der dünnwandigen Membran 7 versehen, die sich bei Auftreten eines Unterdrucks, durch den Pfeil 8 in der Fig. 3 angedeutet, in dem Schlauch 3 in Richtung der Schlauchmitte bewegt, die durch die Längsachse 14 definiert ist. Die Fig. 3 zeigt die Schnittzeichnung durch die Anordnung nach Fig. 1 bei einem solchen Unterdruck 8. Die Membran 7 weist beispielsweise eine Dicke 15 von 0,2 Millimeter auf. Die Verformungen dieser Membran 7 sind in der Fig. 2 zur Verdeutlichung übertrieben dargestellt.

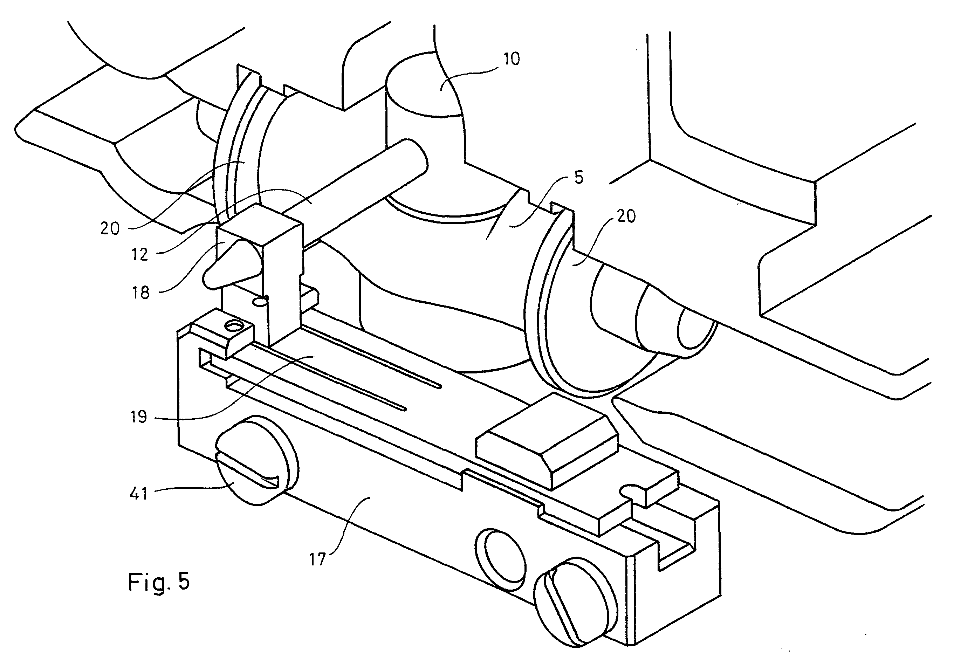

Die Fig. 4 zeigt eine schematische Querschnittsansicht durch den Schlauch 3 im Bereich des Stiftes 12. Der Stift 12 ist in seinem Drehpunkt 13 verankert und kann sich nur um die mit dem Pfeil 16 bezeichnete Achse verschwenken. Damit ist es möglich, eine sehr geringe Bewegung der Membran 7 auf einen Kraftsensor 17 zu übertragen.
Der Stift 12 greift hier in einen Zapfen 18, der die Bewegung auf einen Ausleger 19 des Kraftsensors 17 überträgt.

Die Fig. 5 zeigt ein Ausführungsbeispiel eines Druckmesssystems mit einer solchen Umsetzung nach Fig. 4. Der Schlauchabschnitt 5 endet in zwei Flanschen 20 zum Anschluss eines Leitungssystems. Die Druckmesskammer ist im zusammengezogenen Zustand dargestellt. Die Nase 10 ist zylindrisch. Der metallische Stift 12, der am hier nicht sichtbaren, verdeckten Punkt 13 einseitig eingespannt ist, überträgt als Hebel nun die Bewegung der Membran 7 auf den Kraftsensor 17.

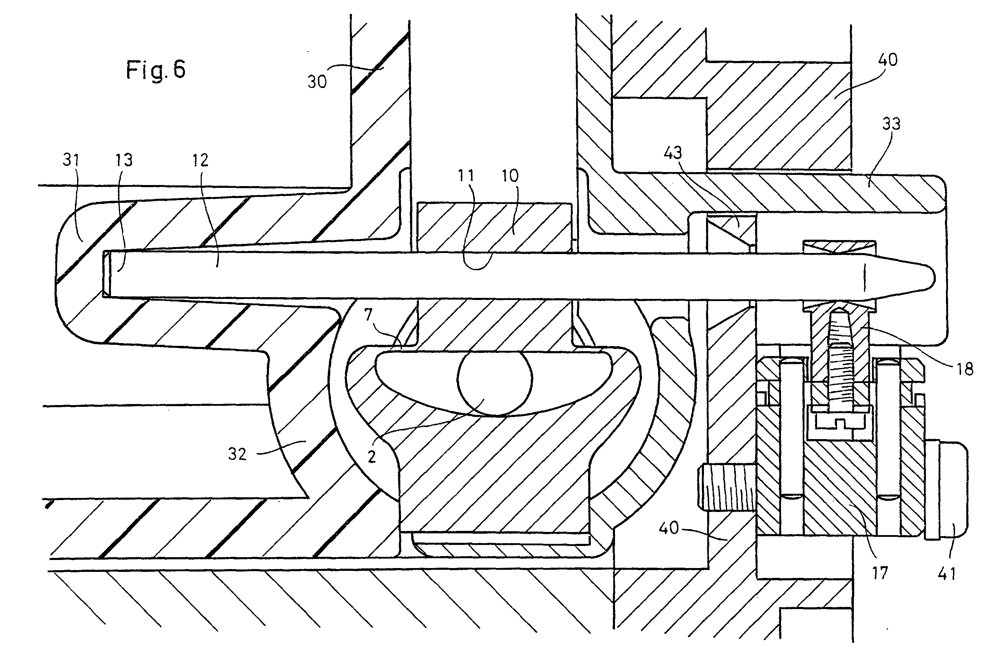

Die Fig. 6 zeigt das Ausführungsbeispiel nach Fig. 5 in einer Schnittansicht. Dabei wird besonders das Einbauprinzip deutlich. Die Druckmesskammer im Schlauchabschnitt 5, der Übertragungsstift 12 und das gesamte Schlauchsystem sind in einer Kassette 30 aus Kunststoff integriert. Zu dieser Kassette 30 gehört auch das Lager 31 des Stiftes 12 und die Ummantelung 32 der Druckmesskammer. Der Drucksensor 17 dagegen ist Bestandteil einer Kassettenaufnahme 40 und über eine Schraubverbindung 41 mit dieser fest verbunden. Beim Einschieben der Kassette 30 in die Kassettenaufnahme 40 wird mittels der Führungsnase 33, die Bestandteil der Kassette 30 ist und die mit dem Führungselement 43 der Kassettenaufnahme zusammenwirkt, der Kopplungsstift 12 sauber zentriert und mit dem Drucksensor 17 verbunden.

## Patentansprüche

1. Druckmesssystem mit einer von einem Fluid (4) durchflossenen Leitung (3), wobei die Leitung (3) einen Leitungsabschnitt (5) aufweist, in dem auf einer abgeflachten Aussenseite (6) der Leitung (3) eine Membran (7) vorgesehen ist, die unlösbar mit einer von der Leitung (3) wegweisenden Nase (10) verbunden ist, die über einen im wesentlichen quer zur longitudinalen Ausrichtung (14) des Leitungsabschnittes (5) ausgerichteten Fortsatz (12) verfügt, dessen für Druckänderungen charakteristische Bewegung (8) an einen Drucksensor (17) übertragbar ist, **dadurch gekennzeichnet, dass** der Fortsatz (12) in einen quer zu ihm angeordneten Zapfen (18) eingreift, mit dem ein einseitig eingespannter Ausleger (19) eines Kraftsensors (17) in mechanischer und zugleich lösbarer Weise als Drucksensor beaufschlagbar ist.

2. Druckmesssystem nach Anspruch 1, **dadurch gekennzeichnet, dass** die besagte Nase (10) eine Bohrung (11) aufweist, durch die der Fortsatz in Gestalt eines Stiftes (12) ragt, der durch mechanischen Kontakt (18, 19) den Kraftsensor (17) in lösbarer Weise kontaktiert.

3. Druckmesssystem nach Anspruch 2, **dadurch gekennzeichnet, dass** der Stift (12) auf der dem Kraftsensor (17) gegenüberliegenden Seite in einem gegenüber der Leitung (3) festen Punkt (13) angelenkt ist.

4. Druckmesssystem nach einem der Ansprüche 1 bis 3, **dadurch gekennzeichnet, dass** der Leitungsabschnitt (5) mit Nase (10) und Fortsatz (12) in einer Kassette (30) integriert ist, dass der Kraftsensor (17) in einer Kassettenaufnahme (40) befestigt ist, dass die Kassette (30) und die Kassettenaufnahme (40) komplementäre Führungselemente (33, 43) aufweisen, so dass bei einem Einsetzen der Kassette (30) in die Kassettenaufnahme (40), der Fortsatz (12) zentriert mit dem Drucksensor (17) verbindbar ist.

5. Druckmesssystem nach Anspruch 4, **dadurch gekennzeichnet, dass** das Führungselement (43) der Kassettenaufnahme (40) eine Bohrung aufweist, durch die der Fortsatz (12) hindurchführbar ist, so dass er in einen Zapfen (18) des Drucksensors (17) eingreift.

## Claims

1. A pressure measuring system having a line (3) through which a fluid (4) flows, the line (3) having a line section (5) in which, on a flattened outer side (6) of the line (3), there is provided a diaphragm (7) being nondetachably connected to a protrusion (10) which points away from the line (3) and has an extension (12) which is oriented substantially transversely with respect to the longitudinal alignment (14) of the line section (5) and whose movement (8), characteristic of pressure changes, can be transmitted to a pressure sensor (17), **characterized in that** the extension (12) engages in a journal (18) which is arranged transversely with respect thereto and with which an extension arm (19) clamped in on one side and belonging to the force sensor (17) can be acted on.

2. The pressure measuring system as claimed in claim 1, wherein the aforesaid protrusion (10) has a hole (11), through which the extension projects in the shape of a pin (12), which, by means of mechanical contact (18, 19), makes contact in a detachable way with the force sensor (17).

3. The pressure measuring system as claimed in claim 2, wherein, on the side opposite the force sensor (17), the pin (12) is attached at a point (13) that is fixed with respect to the line (3).

4. The pressure measuring system as claimed in one of claims 1 to 3, wherein the line section (5) with protrusion (10) and extension (12) is integrated in a cartridge (30), wherein the force sensor (17) is fixed in a cartridge holder (40), wherein the cartridge (30) and the cartridge holder (40) have complementary guide elements (33, 43), so that when the cartridge (30) is inserted into the cartridge holder (40), the extension (12) can be connected in a centered manner to the pressure sensor (17).

5. The pressure measuring system as claimed in claim 4, wherein the guide element (43) of the cartridge holder (40) has a hole through which the extension (12) can be guided, so that it engages in a journal (18) belonging to the pressure sensor (17).

## Revendications

1. Système de mesure de pression comprenant une conduite (3) parcourue par un fluide (4), la conduite (3) présentant une portion de conduite (5) dans laquelle est prévue une membrane (7) sur un côté extérieur aplati (6) de la conduite (3), laquelle est connectée de manière permanente à un nez (10) tourné à l'écart de la conduite (3), lequel dispose d'une projection (12) orientée essentiellement transversalement à l'orientation horizontale (14) de la portion de conduite (5), dont le déplacement caractéristique (8) pour des variations de pression peut être transmis à un capteur de pression (17), **caractérisé en ce que** la projection (12) vient en prise dans un tourillon (18) disposé transversalement à elle, avec lequel un bras tendu d'un côté (19) d'un capteur de force (17) peut être sollicité de manière mécanique et simultanément amovible en tant que capteur de pression.

2. Système de mesure de pression selon la revendication 1, **caractérisé en ce que** ledit nez (10) présente un alésage (11) à travers lequel la projection pénètre sous la forme d'une tige (12) qui vient en contact de manière amovible avec le capteur de force (17) par contact mécanique (18, 19).

3. Système de mesure de pression selon la revendication 2, **caractérisé en ce que** la tige (12) est articulée sur le côté opposé au capteur de force (17) en un point fixe (13) par rapport à la conduite (3).

4. Système de mesure de pression selon l'une quelconque des revendications 1 à 3, **caractérisé en ce que** la portion de conduite (5) avec son nez (10) et sa projection (12) est intégrée dans une cassette (30), **en ce que** le capteur de force (17) est fixé dans un logement de la cassette (40), **en ce que** la cassette (30) et le logement de la cassette (40) présentent des éléments de guidage complémentaires (33, 43), de sorte que dans le cas d'une insertion de la cassette (30) dans le logement de la cassette (40), la projection (12) puisse être connectée de manière centrée au capteur de pression (17).

5. Système de mesure de pression selon la revendication 4, **caractérisé en ce que** l'élément de guidage (43) du logement de la cassette (40) présente un alésage à travers lequel la projection (12) peut être guidée de manière à venir en prise avec un tourillon (18) du capteur de pression (17).
